# EUROPEAN PATENT SPECIFICATION

(11) **EP 2 459 728 B1**
(45) Date of publication and mention of the grant of the patent: **05.06.2013**
(21) Application number: 10742423.6
(22) Date of filing: 23.07.2010
(51) Int. Cl.: C12P 19/32

(54) **Enzymatic synthesis of carba-NAD**
Enzymatische Synthese von Carba-NAD
Synthèse enzymatique de carba-NAD

(30) Priority: 27.07.2009 EP 09166457
(43) Date of publication of application: 06.06.2012
(73) Proprietor: Roche Diagnostics GmbH, 68305 Mannheim (DE); F.Hoffmann-La Roche AG, 4070 Basel (CH)
(72) Inventor: DUEFEL, Hartmut, 82444 Schlehdorf (DE); HEINDL, Dieter, 82396 Paehl (DE); HORN, Carina, 68647 Biblis (DE); MEIER, Thomas, 81373 Muenchen (DE); SCHMUCK, Rainer, 83671 Benediktbeuern (DE)
(74) Representative: Burger, Alexander
(86) International application number: PCT/EP2010/004523
(87) International publication number: WO 2011/012270

(56) References cited:
- WO-A-2007/012494
- SLAMA, J.T. & SIMMONS, A.M.: "Carbanicotinamide Adenine Dinucleotide: Synthesis and Enzymological Properties of a Carbocyclic Analogue of Oxidized Nicotinamide Adenine Dinucleotide" BIOCHEMISTRY, vol. 27, no. 1, 1988, pages 183-193, XP002405653 cited in the application

## Description

### Field of the Invention

The invention concerns the enzymatic synthesis of stable analogues of nicotinamide adenine dinucleotide NAD/NADH and nicotinamide adenine dinucleotide phosphate NADP/NADPH, the so-called "carba-NADs", i.e. analogues of NAD/NADH or NADP/NADPH, respectively, comprising a carbacyclic sugar instead of ribose.

### Background of the Invention

Measuring systems for biochemical analytics are important components of clinically relevant analytical methods. This primarily concerns the measurement of analytes e.g. metabolites or substrates which are determined directly or indirectly with the aid of an enzyme. Frequently an analyte of interest is converted with the aid of an enzyme-coenzyme complex and subsequently quantified via this enzymatic reaction. In this process the analyte to be determined under appropriate reaction conditions is brought into contact with a suitable enzyme and a coenzyme whereby the coenzyme is changed e.g. oxidized or reduced by the enzymatic reaction. This process can be detected electrochemically or photometrically either directly or by means of a mediator. Usually, a calibration curve provides a direct correlation between the measured value and the concentration of the analyte of interest and the analyte concentration can be thereby determined.

Coenzymes are organic molecules which are covalently or non-covalently bound to an enzyme and are changed by the conversion of the analyte. Prominent examples of coenzymes are nicotinamide adenine dinucleotide (NAD) and nicotinamide adenine dinucleotide phosphate (NADP) from which NADH and NADPH, respectively, are formed by reduction.

As described in US 2008/0213809, the disadvantages of conventional measuring systems, e.g., a limited shelf-life, special requirements for storage conditions such as cooling or dry storage in order to achieve improved shelf-life can at least to a great extent be overcome by the stable nicotinamide adenine dinucleotide (NAD/NADH) and nicotinamide adenine dinucleotide phosphate (NADP/NADPH) derivatives disclosed therein. These stable NAD(P)H analogues are appropriate to avoid erroneous results caused by incorrect, unnoticed, faulty storage which is especially important, e.g., in the case of tests which are carried out by the end-users themselves such as in glucose self-monitoring.

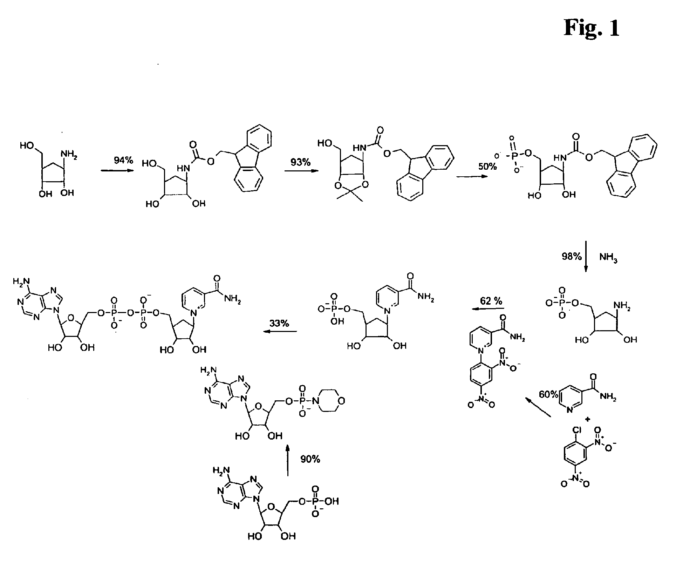

As described in US 2008/0213809 the chemical synthesis of carba-NAD is extremely challenging, requires at least 8 synthesis steps, has rather a low yield and overall thus is quite expensive. The chemical route for synthesis of carba-NAD is depicted in Figure 1. Alternative routes of synthesis are urgently needed.

Hence an object of the present invention is to provide carba-NAD in a less cumbersome manner, with high yields and at attractively low costs.

It has now been surprisingly found that it is possible to utilize enzymes instead of conventional chemistry in order to provide carba-NAD in a cost-effective and convenient manner.

### Summary of the Invention

The present invention relates to a method for enzymatically synthesizing carba-NAD or an analogue thereof the method comprising the steps of a) phosphorylating a 3-Carbamoyl-1-(2,3-dihydroxy-4-hydroxymethyl-cyclopentyl)-pyridinium-methansulfonate or an analogue thereto by aid of an NRK enzyme, b) adenylating the phosphorylated product of step (a) with adenosine or a structurally related compound by aid of an NMN-AT enzyme thereby obtaining carba-NAD or an analogue thereto.

### Detailed Description of the Invention

The present invention relates to a method for synthesis of carba-NAD or an analogue thereof, the method comprising the steps of
a) phosphorylating the compound of Formula I by aid of a nicotinamide ribosyl kinase (NRK) enzyme, 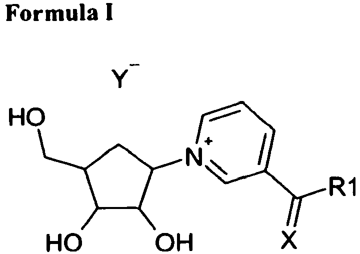 wherein R1 is OH, NH2, O-methyl or N-dimethyl, methyl, Y⁻ is a counter ion and X is O or S,
b) adenylating the phosphorylated product of step (a) with a compound of Formula II by aid of an NMN-AT enzyme. 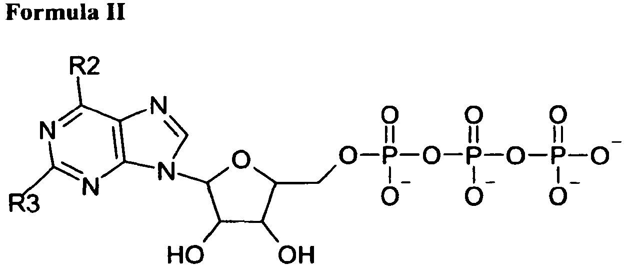 wherein R2 is NH2, OH, or NHalkyl,
   wherein R3 is H, OH, NH2,
   thereby obtaining carba-NAD or an analogue thereof of Formula III. 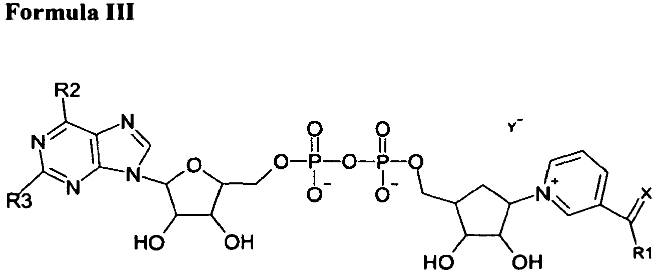
wherein, R1, R2, R3, Y⁻ and X are as defined above.

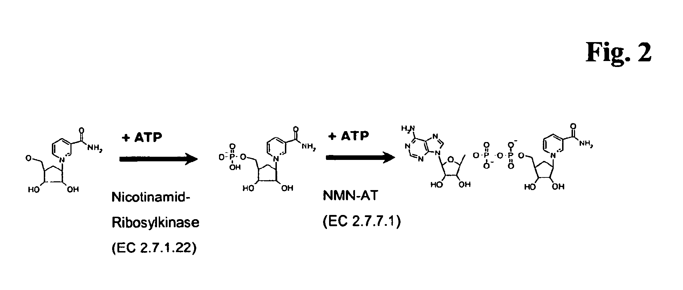

The above method is also illustrated by the reaction scheme shown in Figure 2.

The term "carba-" is used to indicate that instead of a ribosyl sugar residue a 2,3-dihydroxycyclopentane is present. With other words, a carba-analogue of oxidized nicotinamide adenine dinucleotide (NAD'), e.g., is a compound otherwise identical to (NAD') except that a 2,3-dihydroxycyclopentane ring replaces the D-ribonucleotide ring of the nicotinamide riboside moiety (Slama, J.T. and Simmons, A.M., Biochemistry 27 (1988) 1831).

Enzymes are known as highly specific catalyst allowing for reactions to occur at more or less physiological conditions which in their absence would require harsh conditions or would sometimes even be almost impossible to achieve. In order to be able to perform such specific reactions and as a result of evolution through generations and generations under selection pressure enzymes tend to be very specific both with regard to substrate specificity as well as with regard to the reaction catalyzed. It now has been surprisingly found that nicotinamide ribosyl kinases accept the pyridinium compounds of Formula I comprising a 2,3, dihydroxycyclopentane ring instead of a ribosyl residue as a substrate and are capable of phosphorylating these compounds.

Nicotinamide ribosyl kinases (NRKs) according to international enzyme nomenclature are grouped into class EC 2.7.1.22 (ATP:N-ribosylnicotinamide 5'-phosphotransferases). An enzyme chosen from class EC 2.7.1.22 is used in a method according to the present invention in order to phosphorylate a compound of Formula I. Preferred NRKs are those known from *Saccharomyces cerevisiae, Pseudomonas aeruginosa, Streptococcus sanguinius* and *Homo sapiens.* Also preferred the NRKs used in a method according to the present invention are those known from *Streptococcus sanguinius* and *Homo sapiens.* In one preferred embodiment the NRK1 as known from *Homo sapiens* is used in order to perform the first step in a method according to the present invention.

As indicated in Formula I not only carba-nicotinamide with R1 being NH2, but also other compounds like the carba-nicotinamide analogues defined and summarized by the alternatives given for R1 represent an appropriate substrate for certain NRK enzymes. Armed with the disclosure of the present invention the skilled artisan will have no problem to investigate the compounds of Formula I as well as related compounds for their ability to be effectively phosphorylated by an NRK enzyme. The pyridinium compounds as defined in Formula I are used for enzymatic phosphorylation in a method according to the present invention. An analogue to nicotinamide is a compound as defined in Formula I, wherein R1 is not NH2. Preferably R1 of Formula I is selected from the group consisting of OH, NH2 and O-methyl. In one preferred embodiment R1 is OH and in yet one other preferred embodiment R1 is NH2. Alkyl in R1 or R2 preferably is C1 to C6 linear or branched alkyl, preferably linear alkyl.

The residue X in Formula I may be either O or S. In one preferred embodiment X in Formula I is O.

The counter ion Y⁻ preferably is selected from the group consisting of methylsulfonate, Cl⁻ , PF6⁻, BF4⁻, and ClO4⁻. Also preferred the counter ion is BF4⁻or methylsulfonate.

Surprisingly, nicotinamide nucleotide adenylyltransferases (NMN-ATs) can use the phosphorylated carba-nicotinamide obtained as described above as acceptor molecules and are able to adenylate these compounds. In the second step of the enzymatic synthesis of carba-NAD or an analogue thereof a nicotinamide mononucleotide adenylyltransferase is thus used to transfer an adenyl residue or an analogue thereof to the phosphorylated carba-nicotinamide or an analogue thereof, thereby forming carba-NAD or an analogue thereof.

Nicotinamide nucleotide adenylyltransferases (NMN-ATs) according to international enzyme nomenclature are grouped into class EC 2.7.7.1 (ATP:nicotinamide-nucleotide adenylyltransferases). An enzyme chosen from class EC 2.7.7.1 is used in a method according to the present invention in order to adenylate a phosphorylated compound of Formula I with a compound according to Formula II. Preferred NMN-ATs are those known from *Bacillus subtilis, Escherichia coli, Methanococcus janashii, Sulfolobus solfataricus, Saccharomyces cerevisiae* and *Homo sapiens.* In one preferred embodiment the NMN-AT as known from *Homo sapiens,* e.g. expressed in *E. coli* or in *B. subtilis* is used in order to perform the second enzymatic reaction in a method according to the present invention. Despite the fact that not only an adenyl group, but also analogues thereto can be used as a substrate for NMN-AT in a method as disclosed in the present invention and for the sake of convenience the terms adenylate, adenylated or adenylation are used for all these substances unisonous.

It has also surprisingly been observed that both steps in the enzymatic synthesis of carba NAD or an analogue therto can be performed in a single reaction mixture. In yet a further preferred embodiment the present invention relates to a method for enzymatically synthesizing carba-NAD or an analogue thereof the method comprising the steps of a) phosphorylating a 3-Carbamoyl-1-(2,3-dihydroxy-4-hydroxymethyl-cyclopentyl)-pyridinium-methansulfonate or an analogue thereto by aid of an NRK enzyme, b) adenylating the phosphorylated product of step (a) with adenosine or a structurally related compound by aid of an NMN-AT enzyme thereby obtaining carba-NAD or an analogue thereto, wherein both enzymatic reactions are performed in one reaction mixture.

It has surprisingly been found that based on the method disclosed in the present invention the biologically relevant enantiomer of cNAD that is based on the 1R,2S,3R,4R enantiomer of Carbamoyl-1-(2,3-dihydroxy-4-hydroxymethyl-cyclopentyl)-pyridinium can be obtained in pure form and high yield. In a preferred embodiment the method disclosed in the present invention is used to synthesize cNAD comprising the 1R,2S,3R,4R enantiomer of Carbamoyl-1-(2,3-dihydroxy-4-hydroxymethyl-cyclopentyl)-pyridinium.

As indicated in Formula II not only adenosine-tri-phosphate but also other structurally related compounds like the ones characterized and summarized by the definitions given for R2 and R3, respectively, in Formula II. Compounds with the various possible combinations of R2 and R3, respectively, in Formula II represent an appropriate substrate for certain NMN-AT enzymes. Armed with the disclosure of the present invention the skilled artisan will have no problem to investigate the compounds of Formula II as well as structurally related compounds for their ability to be effectively adenylated by an NMN-AT enzyme. A structurally related compound to adenosine is a compound as defined in Formula II, wherein R2 is not NH2 and wherein R3 is not H, respectively. Preferably the purine compounds as defined via the groups given for R2 and R3 in Formula II, respectively, are used for enzymatic adenylation of a phosphorylated carba-nicotinamide or an analogue thereof.

In a further preferred embodiment the present disclosure relates to the use of a compound that is related to a compound of Formula II and selected from the group consisting of the triphosphates of Nebularine, Formycin, aristeromycin, 7 deaza-adenosin, 7 deaza-guanosin, 7 deaza-inosin, 7 deaza-xanthosin, 7 deaza 2,6-diamino purine, 7 deaza 8 aza-adenosin, 7 deaza 8 aza-guanosin, 7 deaza 8 aza-inosin, 7 deaza 8 aza-xanthosin, 7 deaza 8 aza 2,6- diamino purine, 8 aza-adenosin, 8 aza-guanosin, 8 aza-inosin and 8 aza-xanthosin and 8 aza 2,6- diamino purine. These compounds can also be used to produce a corresponding dinucleotide comprising a carba analogue of nicotinamide in a method according to the present disclosure.

Preferably R2 of Formula II is selected from the group consisting of NH2 or OH. In one preferred embodiment R2 is OH and in yet one other preferred embodiment R2 is NH2.

Preferably R3 of Formula II is selected from the group consisting of H or OH. In one preferred embodiment R3 is H.

In one preferred embodiment the method according to the present invention is practiced with the compounds given in Formulas I, II and III, wherein R1 is NH2, R2 is NH2, R3 is H and X is O.

As obvious to the skilled artisan carba-NAD or its analogues, respectively, will not work exactly the same way with the various different enzymes requiring NAD as a co-enzyme or a co-factor. However, the skilled artisan will have no problem to choose the most appropriate analogue out of the options now at stake.

### Description of the Figures

- **Figure 1**: FIG. 1 illustrates in a diagram the standard route used to chemically synthesize carba-NAD (cNAD). As indicated by the percentages given, the total yield according to this procedure is rather low.
- **Figure 2**: FIG. 2 illustrates schematically the two enzymatic steps used in the synthesis of carba-NAD as disclosed in the present invention.

### Example 1: (not according to the invention)

### Synthesis of 5- Dimethy lamino-4-methoxycarbonyl-penta- 2,4-dienylidene-dimethyl-ammoniumtetrafluoroborate

### Example 1.1: Synthesis of Methyl-(2E)-3-(3-dimethylamino)prop-2-enoate

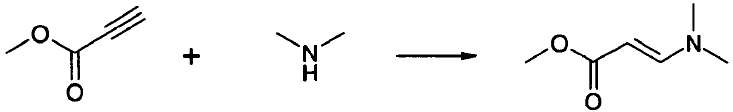

To a solution of methylpropiolate (68.0 ml, 0.764 mol) in 700 ml of dry THF a 2 M solution of N,N-dimethylamine in the same solvent (392 ml, 0.783 mol) was added within 1h at room temperature. After removing the solvent the residue was dried for 1 h (37 °C, 10-20 mbar) at the evaporator resulting a pale yellow solid. The crushed solid was washed with n-hexane to yield 93.0 g (94%) methyl-(2E)-3-(3-dimethylamino)prop-2-enoate that was pure according to TLC and 1H NMR.

### Example 1.2: Synthesis of Pyridiniumtetrafluoroborate

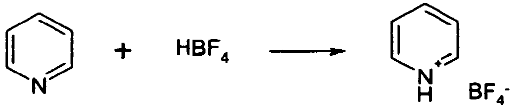

Tetrafluoroboric acid (250 ml, 2.00 mol) was added to cool (0°C) pyridine (157.7 ml, 1.95 mol) within 25 min obtaining a colorless precipitate. After the acid was completely added the mixture was further stirred for 30 min at the same temperature. Then the reaction mixture was filtered. The residue was washed twice with cold ethanol and dried 12 h at high vacuum to yield 201.9 g (60%) pyridiniumtetrafluoroborate as colorless crystals.

### Example 1.3: Synthesis of 5-Dimethylamino-4-methoxycarbonyl-penta-2,4-dienylidene-dimethyl-ammoniumtetrafluoroborate

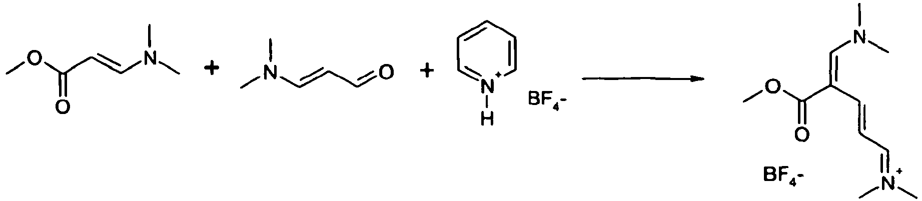

Pyridiniumtetrafluoroborate (283.7 g, 1.70 mol) was added to a solution of methyl-(2E)-3-(3-dimethylamino)prop-2-enoate in 442.5 ml acetic anhydride / acetic acid (2:1). The resulting suspension was cooled to 0°C and 3-dimethylaminoacroleine (169.9 ml, 1.70 mol) was added slowly (3 h) under vigorously stirring and cooling with an ice bath receiving an yellow-brown precipitate. After further stirring for 2 h at room temperature the reaction mixture was filtered. The remaining solid was washed with diethylether several times and dried under reduced pressure. Recrystallization from i-propanol / ethanol (2:1) gave 326.7 g (65%) of the pentamethinium salt as yellow crystals.

### Example 2:

### Synthesis of 2,3-Dihydroxy-4-hydroxymethyl-1-aminocyclopentan

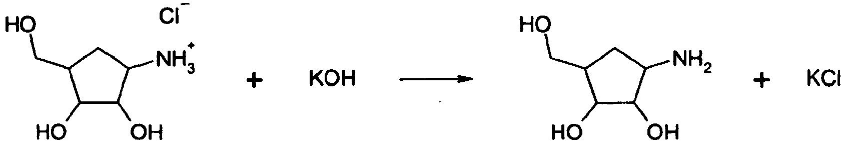

A 1M solution of KOH in EtOH (54.5 ml, 54.5 mmol) was added to a cooled (0°C) solution of the hydrochloride (10.0 g, 54.5 mmol) solved in 540 ml EtOH. After 15 min stirring at room temperature the formed colorless precipitate was removed by filtration. The filtrate was concentrated under reduced pressure. The remaining oil was dried at the evaporator (1 h, 40°C) yielding 9.01 g (112%) of amino carbaribose as pale yellow oil. The obtained product is used for the following steps without further purification.

This procedure is used for synthesis of (1R,2S,3R,4R)-2,3-Dihydroxy-4-hydroxymethyl-1 aminocyclopentan and the enantiomer thereof.

### Example 3:

### Synthesis of 1-(2,3-Dihydroxy-4-hydroxymethyl-cyclopentyl)-3-methoxycarbonyl-pyridinium-methansulfonate

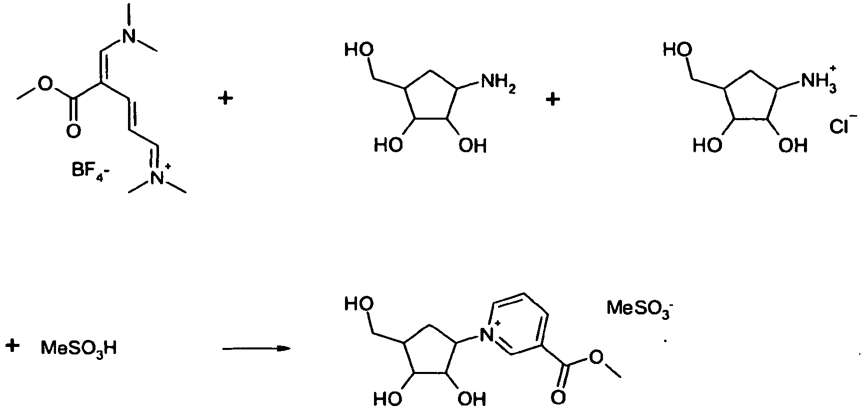

Vinamidinium salt (298.1 g, 1.00 mol) was solved in 1500 ml DMF and I equivalent of methanesulfonic acid (65.02 ml, 1.00 mol) was added. This mixture was dropped continuously and very slowly (within 5 h) to a refluxing solution (90°C) of 3-Amino-5-hydroxymethyl-cyclopentane-1,2-diol (165.3 g, 0.90 mol) and 3-Amino-5-hydroxymethyl-cyclopentane-1,2-diol (25.8 g, 0.15 mol) in 1250 ml MeOH. After the completely addition of the vinamidinium salt solution the reaction mixture was cooled down to room temperature and again 0.15 equivalents methanesulfonic acid were added. The mixture was stirred for 12 h at the same temperature. After removing the solvent under reduced pressure a red-brown oil was obtained, that was further dried for 3 h (45 °C, 4 mbar). Yield: 693.0 g (191%, containing salts and a larger amount of solvent).

This procedure is used for synthesis of 3-methoxycarbonyl-1-((1R,2S,3R,4R)-2,3-dihydroxy-4-hydroxymethyl-cyclopentyl)-pyridinium salt and the enantiomer thereof.

### Example 4:

### 3-Carbamoyl-1-(2,3-dihydroxy-4-hydroxymethyl-cyclopentyl)-pyridinium-methansulfonate

The crude 1-(2,3-Dihydroxy-4-hydroxymethyl-cyclopentyl)-3-methoxycarbonyl-pyridinium-methansulfonate material from Example 3 was rapidly converted into the corresponding amide without further purification.

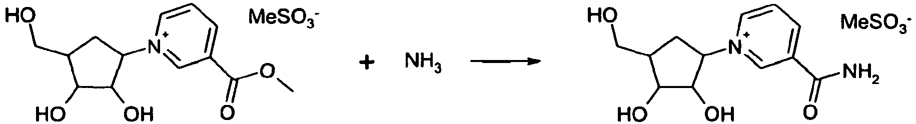

Crude 1-(2,3-Dihydroxy-4-hydroxymethyl-cyclopentyl)-3-methoxycarbonyl-pyridinium-methansulfonate 118.3 g, 173.7 mmol) was dissolved in 100.0 ml methanol. After the addition of methanolic ammonia (7M, 350.0 ml, 2.45 mol) the reaction mixture was stirred for 2.5 h. After removing the solvent under reduced pressure a red-brown oil was obtained that was further dried for 3 h (40 °C, 10 mbar). This crude product is pre-purified with activated charcoal and can e.g. be used directly for the chemical synthesis of cNAD (WO2007/012494) or in the enzymatic synthesis of cNAD as described herein below.

Other compounds appropriate for use in a method according to the present invention, see e.g. the compounds defined in Formula I, can be synthesized in a manner analogous to the procedures given in Examples 1 to 4 herein above.

This procedure is used for synthesis of 3-Carbamoyl-1-((1 R,2S,3R,4R)-2,3-dihydroxy-4-hydroxymethyl-cyclopentyl)-pyridinium salt and the enantiomer thereof.

### Example 5:

### Enzymatic phosphorylation of several compounds according to Formula I with nicotinamide ribosyl kinase

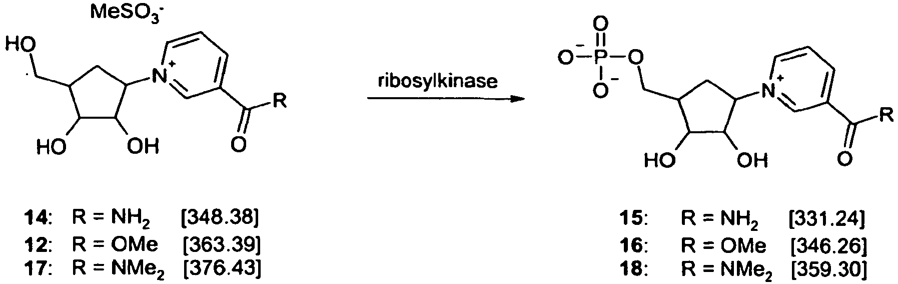

| | |
|---|---|
| Pure (1R,2S,3R,4R) enantiomers of **12**, **14**, **17,** | |
| respectively, 100 mg/ml: | 100 µl |
| TRIS x HCl buffer pH 7.5, 15 mM MgCl₂: | 960 µl |
| ATP solution 100 mM/1: | 40 µl |
| creatine phosphate: | 14.5 mg |
| creatine kinase: | 0.1 mg |
| nicotinamide ribosyl kinase, 0.7 U/ml | 230 µl |

(Recombinant NRK1 from *Homo sapiens* (SwissProt ID: Q9NWW6) or NRK (nadR) from *S. sanguinis* (SwissProt ID: A3CQV5), expressed heterologous in *E*. *coli*).

### General working procedure:

Creatine phosphate (14.5 mg) and creatine kinase (0.1 mg) were dissolved in a mixture of TRIS buffer (pH 7.5, 15 mM MgCl₂, 960 µl) and ATP (100 mM/1 in H₂O, 40 µl). Then a solution of the riboside (compound **14** or analogue as given above) (100 mg/ml in H₂O, 100 µl) followed by ribosyl kinase (0.7 U/ml, 230 µl) was added. The reaction mixture was incubated 16 h at 37°C. After a short warm up on 80 °C the mixture was filtered and investigated by HPLC.

In all three cases (with compounds **14, 12** or **17**) the complete consumption of the riboside and the formation of a new compound (the corresponding phosphorylated product given as compounds **15, 16** or **18**, **respectively**, **above**) could be detected by HPLC.

Correct masses of the desired phosphorylated products were found via LC/MS: (MS: ESI: M⁻ = 330.75 (compound **15**), 345.74 (compound **16**), 358.79 (compound **18**)).

**Compound 15** is purified by using chromatography on a cation exchange resin Dowex and eluation with water.

### Example 6:

### Enzymatic conversion of carba-nicotinamide and analogues thereof, respectively, with NMN-AT

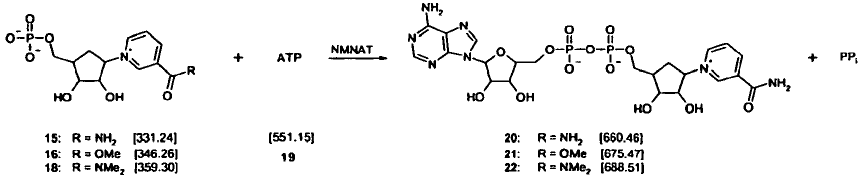

| | |
|---|---|
| **From substances 15, 16 and 18, respectively,** (crude material from enzymatic phosphorylation of example 5) ca. | 10.0 mg |
| substance **19** (adenosinetriphosphate, disodium salt) | 22.6 mg |
| NMN-AT:, (32 U/ml) | 4.8µl (0.153 U) |

(Recombinant nicotinamide mononucleotide-adenosyltranferase (NMN-AT) from *Homo sapiens* (SwissProt ID: Q9HAN9). Alternatively, e.g., NMN-AT from *E*. *coli* (SwissProt ID: P0A752) or *B. subtilis* (SwissProt ID: P54455) expressed heterologous in *E*. *coli is used*.)

### Working procedure:

ATP disodium salt (22.6 mg) and nicotinamide mononucleotide adenosyltranferase (NMN-AT, 4.8 µl, 0.153 U) were added to the filtered solution obtained from the enzymatic phosphorylation containing mononucleotide (compound **15** or an analogue, e.g. compounds 16 and 18). The reaction mixture was incubated 18 h at 37°C. After a short warm up on 80 °C the mixture was filtered and investigated by HPLC und LC/MS.

In all three experiments the complete consumption of the mononucleotide (compounds **15**, **16** or **18**) and the formation of a new compound could be detected by HPLC.

Correct mass of compound **20** was found (MS: ESI: M⁻ = 659.77).

### Example 7:

### One pot procedure for conversion of 3-Carbamoyl-1-((1R,2S,3R,4R)-2,3-dihydroxy-4-hydroxymethyl-cyclopentyl)-pyridinium salt to carba-nicotinamide

1 g (2.16 mmol) of 3-Carbamoyl-1-((1R,2S,3R,4R)-2,3-dihydroxy-4-hydroxymethyl-cyclopentyl)-pyridinium; chloride, 0.242 g (0.4 mmol) ATP di sodium salt, 300 mg Mg C12 x 6H2O (1.45 mmol) 16 U ribosyl kinase, 1.45 g (4.43 mmol) creatinphosphate and 4.27 kU creatin kinase were dissolved in 25 ml sterile water. The mixture was incubated at 35 °C overnight. Then 2.42 g (4 mmol) ATP di sodium salt, 440 mg MgC12 x 6H2O (2.16 mmol) and 32 U NMNAT were added. The mixture was incubated at 35 °C overnight. Than it was heated to 90 °C for 5 min and after cooling filtrated. Purification was performed by using ion exchange chromatography as described in WO 2007/012494.

### Example 8:

### Conversion of 3-Carbamoyl-1-((1R,2S,3R,4R)-2,3-dihydroxy-4-hydroxymethyl-cyclopentyl)-pyridinium salt to carba-nicotinamide in the presence of the enantiomer 3-Carbamoyl-1-((1S,2R,3S,4S))-2,3-dihydroxy-4-hydroxymethyl-cyclopentyl)-pyridinium salt

1 g (2.16 mmol) of 1: 1 mixture consisting of 3-Carbamoyl-1-((1R,2S,3R,4R)-2,3-dihydroxy-4-hydroxymethyl-cyclopentyl)-pyridinium; chloride and 3-Carbamoyl-1-((S,2R,3S,4S)-2,3-dihydroxy-4-hydroxymethyl-cyclopentyl)-pyridinium; chloride 0.242 g (0.4 mmol), ATP di sodium salt, 300 mg Mg Cl2 x 6H2O (1.45 mmol) 16 U ribosyl kinase, 1.45 g (4.43 mmol) creatinphosphate and 4.27 kU creatin kinase were dissolved in 25 ml sterile water. The mixture was incubated at 35 °C overnight. The reaction was monitored by reversed phase HPLC analysis (ODS Hypersil, 5µm, 250 x 4,6 mm Thermo Scientific, Part-Nr.:30105-254630, eluent A = 0.1 M triethylammoniumacetate pH 7.0, eluent B = 0.2 L 0.1 M triethylammmoniumacetate. pH 7.0 + 0.8 L acetonitrile, gradient 2 min 0% B, in 23 min 100 % B, flow: 1 ml / min, detection: UV/ 260 nm) which shows that both enantiomers were phosphorylated. The peak corresponding to 3-Carbamoyl-1-((1R,25,3R,4R)-2,3-dihydroxy-4-hydroxymethyl-cyclopentyl)-pyridinium; chloride and the (1S,2R,3S,4S) enantiomer at 2.96 min disappears and a new peak corresponding to the phosphorylated products at 3.45 min appears.

Then 2.42 g (4 mmol) ATP di sodium salt, 440 mg MgCl2 x 6H2O (2.16 mmol) and 32 U NMN-AT were added. The mixture was incubated at 35 °C overnight. Thereafter it was heated to 90 °C for 5 min and after cooling filtrated. Reversed phase HPLC analysis shows a peak at 7.92 min corresponding to cNAD. At 3.45 min a peak remains which corresponds to the phosphorylated (1S,2R,3S,4S) enantiomer. Upon adding alkaline phosphatase the peak at 7.92 is not influenced whereas the peak of the phosphorylated (1S,2R,3S,4S) enantiomer at 3.45 min disappears and a peak at 2.96 min appears which corresponds to the 3-Carbamoyl-1-((1S,2R,3S,4S)-2,3-dihydroxy-4-hydroxymethyl-cyclopentyl)-pyridinium salt. Thus cNAD (based on the 1R,2S,3R,4R enantiomer) is not effected, whereas the remaining phosphorylated (1S,2R,3S,4S) enantiomer is de-phosphorylated by alkaline phosphatase.

As a controll the same experiment was performed only using the 3-Carbamoyl-1-((1S,2R,3S,4S)-2,3-dihydroxy-4-hydroxymethyl-cyclopentyl)-pyridinium; chloride and monitored by HPLC. There was formation of a peak at 3.45 min (corresponds to the phosphorylated enantiomer) upon adding the Ribosyl kinase but no peak with a retention time at 7.92 min was found in the HPLC chromatogram after adding the NMN-AT.

Therefore it possible to start the synthesis of cNAD with an enantiomeric mixture of 2,3-Dihydroxy-4-hydroxymethyl-1-aminocyclopentan consisting of (1R,2S,3R,4R and 1S,2R,3S,4S) enantiomers and to obtain, by the method disclosed in the present invention solely the biologically relevant cNAD.

Then 2.42 g (4 mmol) ATP di sodium salt, 440 mg MgCl2 x 6H2O (2.16 mmol) and 32 U NMN-AT were added. The mixture was incubated at 35 °C overnight. Thereafter it was heated to 90 °C for 5 min and after cooling filtrated. HPLC analysis shows a peak at 7.92 min corresponding to cNAD. At 3.45 min a peak remains which corresponds to the phosphorylated (1S,2R,3S,4S) enantiomer. Upon adding alkaline phosphatase the peak at 7.92 is not influenced whereas the peak of the phosphorylated (1S,2R,3S,4S) enantiomer at 3.45 min disappears and a peak at 2.96 min appears which corresponds to the 3-Carbamoyl-1-((1S,2R,3S,4S)-2,3-dihydroxy-4-hydroxymethyl-cyclopentyl)-pyridinium salt. Thus cNAD (based on the 1R,2S,3R,4R enantiomer) is not effected, whereas the remaining phosphorylated (1S,2R,3S,4S) enantiomer is de-phosphorylated by alkaline phosphatase.

As a control the same experiment was performed only using the 3-Carbamoyl-1-((1S,2R,3S,4S)-2,3-dihydroxy-4-hydroxymethyl-cyclopentyl)-pyridinium; chloride and monitored by HPLC. There was formation of a peak at 3.45 min (corresponds to the phosphorylated enantiomer) upon adding the Ribosyl kinase but no peak with a retention time at 7.92 min was found in the HPLC chromatogram after adding the NMN-AT.

Therefore it possible to start the synthesis of cNAD with an enantiomeric mixture of 2,3-Dihydroxy-4-hydroxymethyl-1-aminocyclopentan consisting of (1R,2S,3R,4R and 1S,2R,3S,4S) enantiomers and to obtain, by the method disclosed in the present invention, cNAD solely based on the biologically relevant 1R,2S,3R,4R enantiomer.

### Example 9:

### Enzymatic conversion of carba-nicotinamide mononucleotide (substance 15) with NMN-AT and N6 hexylamino ATP

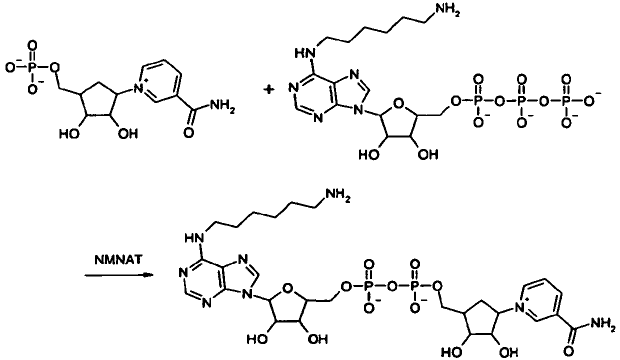

### General working procedure:

N6-hexylaminoATP disodium salt Jena Bioscience (0.33 mg) and nicotinamide mononucleotide adenosyltranferase (NMN-AT, 4.8 µl, 0.153 U) were added to a solution of 1 mg **15.** The reaction mixture was incubated 18 h at 37°C. After a short warm up on 80 °C the mixture was filtered and investigated by HPLC und LC/MS.

Carba-NMN (compound **15**) was completely consumed and new compound (the corresponding adenosyl derivative) was detected by HPLC.

Correct mass was found (MS: ESI: M⁻ = 759.77)

## Claims

1. A method for synthesis of carba-NAD or an analogue thereof, the method comprising the steps of
a) phosphorylating the compound of Formula I by aid of a nicotinamide ribosyl kinase (NRK) enzyme 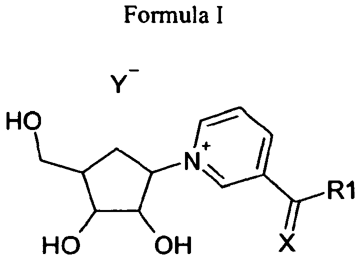 wherein R1 is OH, NH2, O-methyl or N-dimethyl, methyl, Y⁻ is a counter ion and X is O or S,
b) adenylating the phosphorylated product of step (a) with a compound of Formula II by aid of a nicotinamide mononucleotide adenosyltranferase (NMN-AT) enzyme 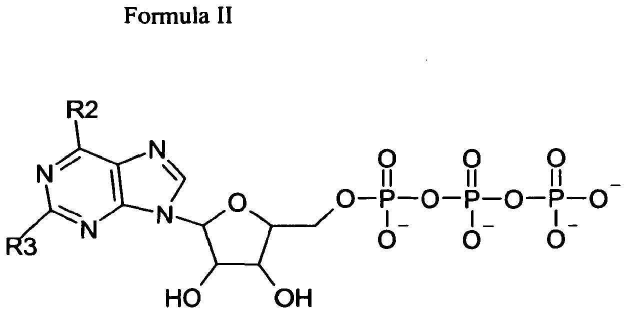 wherein R2 is NH2, OH, or NHalkyl,
wherein R3 is H, OH or NH2,
thereby obtaining carba-NAD or an analogue thereof of Formula III 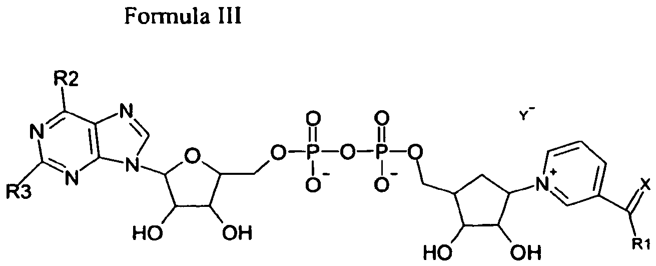 wherein, R1, R2, R3, Y⁻ and X are as defined above.

2. The method of claim 1, wherein said NRK enzyme is selected from NRKs known from *Saccharomyces cerevisiae, Pseudomonas aeruginosa, Streptococcus sanguinius* and *Homo sapiens.*

3. The method of claim 1 or 2, wherein said NMN-AT is selected from the group consisting the NMN-ATs as known from *Bacillus subtilis, Escherichia coli, Methanococcus janashii, Sulfolobus solfataricus, Saccharomyces cerevisiae* and *Homo sapiens.*

4. The method according to any of claims 1 to 3, wherein in the compound of Formula I R1 is selected from the group consisting of OH, NH2 and O-methyl.

5. The method according to any of claims 1 to 3, wherein in the compound of Formula II R2 is NH2 or OH.

6. The method according to any of claims 1 to 3, wherein in the compound of Formula II R3 is H or OH.

7. The method according to any of claims 1 to 3, wherein in the compound of Formula I X is O.

8. The method according to any of the proceeding claims, wherein R1 is NH2, R2 is NH2, R3 is H and X is O.

## Patentansprüche

1. Verfahren für die Synthese von carba-NAD oder einem Analog davon, wobei das Verfahren die folgenden Schritte umfasst:
(a) Phosphorylieren der Verbindung der Formel I mit Hilfe einer Nicotinamidribosylkinase (NRK) 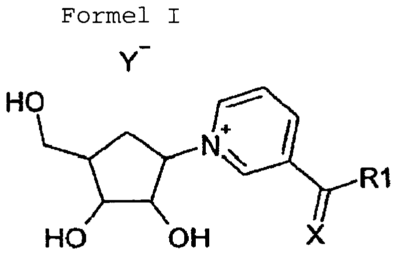 worin R1 OH, NH2, O-Methyl oder N-Dimethyl, Methyl bedeutet, Y⁻ ein Gegenion bedeutet und X O oder S bedeutet,
(b) Adenylieren des phosphorylierten Produkts von Schritt (a) mit einer Verbindung der Formel (II) mit Hilfe einer Nicotinamidmononukleotid-Adenosyltransferase (NMN-AT) 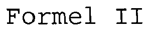 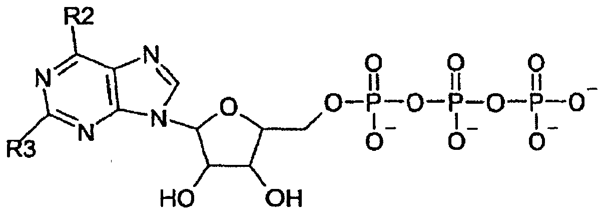 worin R2 NH2, OH oder NH-Alkyl bedeutet,
worin R3 H, OH oder NH2 bedeutet,
wodurch man zu carba-NAD oder einem Analog davon der Formel III 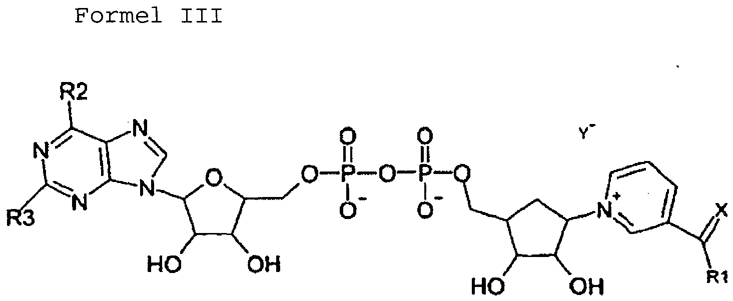 worin R1, R2, R2, Y⁻ und X wie oben definiert sind,
gelangt.

2. Verfahren nach Anspruch 1, wobei die NRK aus der Reihe der aus *Saccharomyces cerevisiae, Pseudomonas aeruginosa, Streptococcus sanguinius* und *Homo sapiens* bekannten Nicotinamidribosylkinasen stammt.

3. Verfahren nach Anspruch 1 oder 2, wobei die NMN-AT aus der Gruppe bestehend aus der aus *Bacillus subtilis, Escherichia coli, Methanococcus janashii, Sulfolobus solfataricus, Saccharomyces cerevisiae* und *Homo sapiens* bekannten Nicotinamidmononukleotid-Adenosyltransferasen stammt.

4. Verfahren nach einem der Ansprüche 1 bis 3, wobei in der Verbindung der Formel I R1 aus der Gruppe bestehend aus OH, NH2 und 0-Methyl ausgewählt ist.

5. Verfahren nach einem der Ansprüche 1 bis 3, wobei in der Verbindung der Formel II R2 NH2 oder OH bedeutet.

6. Verfahren nach einem der Ansprüche 1 bis 3, wobei in der Verbindung der Formel II R3 H oder OH bedeutet.

7. Verfahren nach einem der Ansprüche 1 bis 3, wobei in der Verbindung der Formel I X 0 bedeutet.

8. Verfahren nach einem der vorhergehenden Ansprüche, wobei R1 NH2 bedeutet, R2 NH2 bedeutet, R3 H bedeutet und X O bedeutet.

## Revendications

1. Procédé de synthèse du carba-NAD ou d'un de ses analogues, le procédé comprenant les étapes dans lesquelles :
a) on soumet à une phosphorylation le composé répondant à la formule I à l'aide d'une enzyme de nicotinamide ribosyl kinase (NRK) 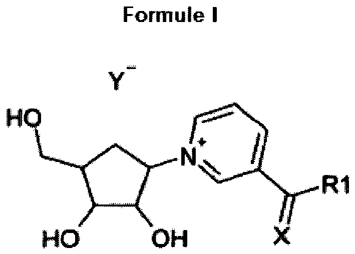 dans laquelle R1 représente un groupe OH, un groupe NH2, un groupe O-méthyle ou un groupe N-diméthyle, un groupe méthyle, Y⁻représente un ion antagoniste et X représente un oxygène ou un atome de soufre ;
b) on soumet à une adénylation le produit phosphorylé de l'étape (a) avec un composé répondant à la formule Il à l'aide d'une enzyme de nicotinamide mononucléotide adénosyltransférase (NMN-AT) 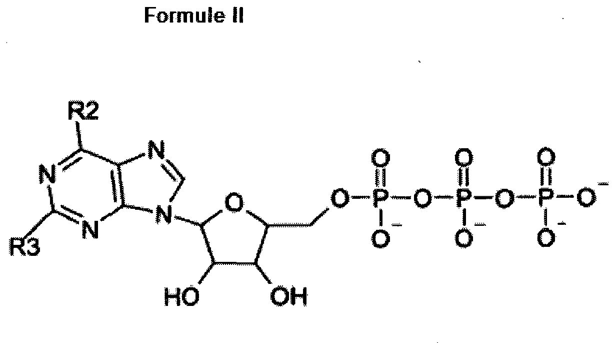 dans laquelle R2 représente un groupe NH2, un groupe OH ou un groupe NHalkyle ;
dans laquelle R3 représente un atome d'hydrogène, un groupe OH ou un groupe NH2 ;
pour ainsi obtenir un carba-NAD ou un de ses analogues répondant à la formule III 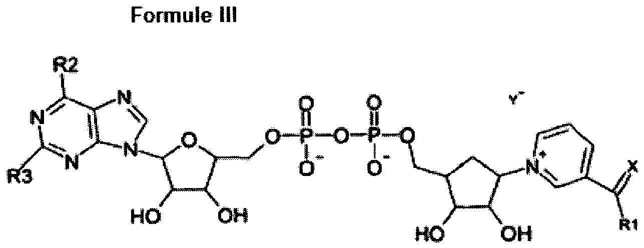 dans laquelle R1, R2, R3, Y⁻ et X sont tels que définis ci-dessus.

2. Procédé selon la revendication 1, dans lequel ladite enzyme NRK est choisie parmi des NRK telles qu'on les connaît de *Saccharomyces cerevisiae*, *Pseudomonas aeruginosa*, *Streptococcus sanguinius* et *Homo sapiens.*

3. Procédé selon l'obligation 1 ou 2, dans lequel ladite NMN-AT est choisie parmi le groupe constitué par les NMN-AT telles qu'on les connaît de *Bacillus subtilis, Escherichia coli, Methanococcus janashii, Sulfolobus solfataricus*, *Saccharomyces cerevisiae* et *Homo sapiens.*

4. Procédé selon l'une quelconque des revendications 1 à 3, dans lequel, dans le composé répondant à la formule I, R1 est choisi parmi le groupe constitué par un groupe OH, un groupe NH2 et un groupe O-méthyle.

5. Procédé selon l'une quelconque des revendications 1 à 3, dans lequel, dans le composé répondant à la formule II, R2 représente un groupe NH2 ou un groupe OH.

6. Procédé selon l'une quelconque des revendications 1 à 3, dans lequel, dans le composé répondant à la formule II, R3 représente un atome d'hydrogène ou un groupe OH.

7. Procédé selon l'une quelconque des revendications 1 à 3, dans lequel, dans le composé répondant à la formule I, X représente un atome d'oxygène.

8. Procédé selon l'une quelconque des revendications précédentes, dans lequel R1 représente un groupe NH2, R2 représente un groupe NH2, R3 représente un atome d'hydrogène et X représente un atome d'oxygène.
